**Europäisches Patentamt**

**European Patent Office**

**Office européen des brevets**

(19)

(11) Numéro de publication : **0 297 973 B1**

# FASCICULE DE BREVET EUROPEEN

(12)

(45) Date de publication du fascicule du brevet :
22.04.92 Bulletin 92/17

(51) Int. Cl.$^5$ : **C09D 5/22**

(21) Numéro de dépôt : **88401649.4**

(22) Date de dépôt : **28.06.88**

(54) **Procédé de contrôle du revêtement d'articles.**

(30) Priorité : **01.07.87 FR 8709304**

(43) Date de publication de la demande :
**04.01.89 Bulletin 89/01**

(45) Mention de la délivrance du brevet :
**22.04.92 Bulletin 92/17**

(84) Etats contractants désignés :
**BE CH DE ES FR GB IT LI NL SE**

(56) Documents cités :
**EP-A- 0 116 273**
**DE-A- 3 151 012**
**DE-A- 3 208 186**

(73) Titulaire : **SAINT-GOBAIN EMBALLAGE**
**Les Miroirs 18, Avenue d'Alsace**
**F-92400 Courbevoie (FR)**

(72) Inventeur : **Martin, Monique**
**37 bis, rue des Chasseurs**
**F-91700 Ste Geneviève des Bois (FR)**

(74) Mandataire : **de Toytot, Robert et al**
**SAINT-GOBAIN RECHERCHE 39 quai Lucien**
**Lefranc B.P. 135**
**F-93304 Aubervilliers Cédex (FR)**

EP 0 297 973 B1

Jouve, 18, rue Saint-Denis, 75001 PARIS

## Description

La présente invention concerne le revêtement d'objets par des couches difficilement observables à la lumière par l'oeil humain.

Elle vise plus particulièrement à contrôler, et éventuellement à maîtriser, la présence et de préférence l'épaisseur et l'homogénéité de telles couches.

Elle intéresse tout spécialement la fabrication des récipients en verre, tels que bouteilles, flacons ou pots, qui doivent résister à de nombreuses manipulations lors de leur fabrication et de leur emplissage, et qui à cet effet font l'object, à la suite de la recuisson, de traitements dits "bout froid".

De tels traitements consistent généralement à déposer sur les articles des revêtements visant à améliorer les propriétés de surface du verre, notamment le glissement et la résistance à l'accroc, sans altérer son brillant et, plus généralement, son aspect.

On utilise couramment, pour constituer de tels revêtements, des cires ou des vernis, déposés par pulvérisation de solutions ou d'émulsions, au trempé, etc...

Sauf dans le cas où un excès de produit à la surface de l'article provoque une altération de son aspect, il n'existait pas de moyens commodes, avant l'invention, pour assurer efficacement, sur la chaîne de fabrication, le contrôle des traitements "bout froid". On se contentait donc généralement d'effectuer des prélèvements sur la ligne, et de déterminer sur les articles ainsi prélevés des caractéristiques telles que l'angle de glissement et la résistance à l'accroc.

De telles méthodes de contrôle présentent de nombreux inconvénients. Elles sont de caractère destructif et donc obligatoirement statistique, et ne se prêtent donc pas à un tri fiable des articles traités. En outre, elles ne permettent pas de détecter avec précision l'instant d'apparition et souvent, en conséquence, l'origine d'un défaut, ce qui retarde les opérations visant à sa correction, nuisant ainsi gravement au rendement de la fabrication, et en obérant le coût.

la présente invention, qui vise à éviter ou tout au moins réduire ces inconvénients, se fonde sur l'incorporation dans le produit de revêtement d'un composé lui conférant des propriétés de fluorescence sous irradiation ultra-violette, tout en ne présentant pas de coloration notable dans les conditions courantes d'éclairement (lumière du jour, ou éclairages artificiels usuels).

L'invention a, très généralement, pour objet un procédé de revêtement d'articles par une ou plusieurs couches de produits de revêtement non visibles dans les conditions usuelles d'éclairement, selon lequel, afin de procéder à un tri des articles tenant compte d'une caractéristique d'au moins l'une des dites couches, telle que son épaisseur, et/ou à un réglage du processus de revêtement en vue de l'obtention de la caractéristique souhaitée, on incorpore de manière homogène au produit de revêtement correspondant à la dite couche, avant son dépôt, au moins un agent de marquage non visible dans les conditions usuelles d'éclairement, mais présentant une fluorescence sous irradiation on soumet les articles, après le dépôt de ladite couche, à une irradiation , et on utilise le signal émis, pour chaque article, par ladite couche contenant l'agent de marquage, sous l'effet de l'irradiation, pour déterminer les caractéristiques de répartition de ladite couche, et procéder au tri desdits articles, et/ou au réglage du processus de formation de ladite couche sur les articles soumis ultérieurement au revêtement.

L'invention a notamment pour objet l'application de ce procédé aux traitements "bout froid" de récipients en verre, par exemple visant à améliorer le glissement et la résistance aux chocs, dans lequel un agent de marquage tel que du 2-5 bis (5 terbutylbenzoxazolyl-2') thiophène est incorporé au produit de traitement bout froid".

L'invention trouve également à s'appliquer avantageusement pour le traitement du bord supérieur du buvant des récipients par une préparation favorisant le thermo-scellage de leur opercule d'obturation : un agent fluorescent sous rayonnement de haute énergie est introduit dans la préparation, et le buvant est contrôlé sous un tel rayonnement après traitement, permettant la détection des lacunes ou autres imperfections du dépôt, et leur correction, le cas échéant.

L'emploi de substances fluorescentes a déjà été décrit comme moyens discrets de marquage, notamment par le document EP-A-253.543, qui vise l'authentification de documents, l'encre d'imprimerie comportant une telle substance, ou par le document EP-A-116.273, qui prévoit l'apposition d'une "marque" invisible en lumière naturelle ou similaire, sur des textiles de prix, tels que tapisseries, pour faire échec aux voleurs, de même que le document DE-A-3151 012 perfectionne un tel "marquage", visible seulement en lumière ultra-violette, pour toutes sortes d'objets de valeur.

Cependant aucun de ces documents ne s'intéresse au véhicule du pigment fluorescent, mais seulement à l'absence ou la présence de la marque, alors que selon l'invention, l'agent fluorescent est utilisé comme moyen intermédiaire pour des déterminations visant la couche qui le contient, l'information obtenue étant utilisée pour un tri des articles revêtus, et/ou de préférence pour le réglage du processus de dépôt de la couche.

Divers exemples sont donnés ci-après pour illustrer l'invention et ses avantages.

Exemple 1 - Cet exemple concerne la fabrication mécanique de bouteilles, et plus particulièrement le traitement "bout froid" appliqué aux bouteilles en sortie d'arche de recuisson.

Dans une cire de polyéthylène additivée de poids moléculaire d'environ 2100, usuelle pour cette application, du type commercialisé par la Société Franqaise Polychimie sous la référence EN 62, mise en dispersion aqueuse à la concentration de 20 %, a été incorporée, à la concentration de 5 %, du 2-5 bis (5 terbutylbenzoxazolyl-2') thiophène, sous la formulation commercialisée par CIBA sous la dénomination UVITEX OB, qui possède des propriétés de fluorescence sous irradiation ultraviolette. Cette nouvelle formulation est dénoie EN 72.

Des dispersions aqueuses de cette préparation, à des dilutions de 1 et 2 % ont été pulvérisées pour un traitement "bout froid" de bouteilles à bière de 26,5 cl selon les méthodes usuelles.

l'observation des bouteilles traitées, effectuée en chambre noire sous irradiation par deux types de lampes UV de forte puissance, émettant un rayonnement de longueur d'onde respectivement 365 nm et 254 nm, permet, pour chacune d'elle une visualisation très nette et précise du revêtement, qui émet une fluorescence violette, les manques, les altérations du dépôt (rayure, frottement) se trouvant parfaitement mis en évidence dans tous les cas, pour les épaisseurs de revêtement usuellement mises en oeuvre.

Le tri des articles peut être effectué manuellement en bout de ligne à l'occasion d'un examen visuel en chambre noire sur irradiation UV. Il a été également effectué avec succès par une machine automatique de contrôle continu dans laquelle un capteur analyse le signal émis par l'article soumis au rayonnement d'excitation lors d'une rotation complète autour de son axe de symétrie.

Selon une forme avantageuse de mise en oeuvre de l'invention, le procédé de dépôt du revêtement comporte un asservissement du débit de la pulvérisation de produit de revêtement au signal émis par le revêtement.

Il est à noter que d'autres techniques d'applications que la pulvérisation, telles que le trempé ou le rouleau, voire l'enduction, sont compatibles avec les propriétés rhéologiques de la dispersion.

Exemple 2 - Une autre variante de mise en oeuvre de l'invention se situe dans la fabrication de pots à thermo-sceller : le rebord supérieur du buvant du récipient étant avantageusement revêtu d'une préparation favorisant le thermo-scellage, on introduit dans cette préparation un agent fluorescent, de manière à détecter de façon simple, les lacunes du traitement, qui sont susceptibles d'invalider le scellement.

Le composé cité plus haut à titre d'agent fluorescent n'était pas utilisé jusqu'à ce jour à ce titre en addition dans les matières plastiques, mais plutôt comme azurant optique de ces matières, agréé pour le contact avec les produits alimentaires. Dans la mise en oeuvre selon l'invention, ce composé s'avère donc présenter un ensemble de propriétés jouant deux fonctions tout à fait favorables au but visé : en tant qu'azurant optique, sa présence ajoute encore à la discrétion du revêtement, tandis que ses propriétés de fluorescence permettent de résoudre les problèmes de contrôle.

## Revendications

1. Procédé de revêtement d'articles par une ou plusieurs couches de produits de revêtement non visibles dans les conditions usuelles d'éclairement, **caractérisé en ce que**, afin de procéder à un tri des articles tenant compte d'une caractéristique d'au moins l'une des dites couches, telle que son épaisseur, et/ou à un réglage du processus de revêtement en vue de l'obtention de la caractéristique souhaitée, on incorpore de manière homogène au produit de revêtement correspondant à la dite couche, avant le dépôt de la dite couche, au moins un agent de marquage non visible dans les conditions usuelles d'éclairement, mais présentant une fluorescence sous irradiation, et en ce qu'on soumet les articles, après le dépôt de la dite couche, à une irradiation, et on utilise le signal émis, pour chaque article, par la dite couche contenant l'agent de marquage, sous l'effet de l'irradiation, pour déterminer les caractéristiques de répartition de la dite couche, et procéder au tri des dits articles, et/ou au réglage du processus de formation de la dite couche sur les articles soumis ultérieurement au revêtement.

2. Applications du procédé selon la revendication 1 aux traitements "bout froid" de récipients en verre par une composition de traitement contenant une cire de polyéthylène additivée de poids moléculaire d'environ 2100, **caractérisé en ce que** du 2-5 bis (5 terbutylbenzoxazolyl-2') thiophène est incorporé à la dite cire.

3. Application du procédé selon la revendication 1 au traitement du bord supérieur du buvant des récipients par une préparation favorisant le thermo-scellage de leur opercule d'obturation, **caractérisé en ce que** un agent fluorescent est introduit dans ladite préparation de traitement et en ce que ledit bord supérieur est examiné sous rayonnement ultra-violet pour le contrôle dudit traitement, la détection des lacunes ou autres imperfections éventuelles, et leur correction le cas échéant, étant ensuite effectuées.

## Patentansprüche

1. verfahren zum Überziehen von Gegenständen mit einer oder mehreren Schichten aus unter normalen Lichtverhältnissen nicht sichtbaren Überzugsstoffen, **dadurch gekennzeichnet**, daß man, um die Gegenstände unter Berücksichtigung eines Kennwerts, beispielsweise der Dicke, wenigstens einer der genamten Schichten zu sortieren, und/oder um den Beschichtungsvorgang im Hinblick auf das Erzielen eines erwünschten Merkmals zu regeln, vor dem Aufbringen der Schicht auf homogene Weise wenigstens einen bei gewöhnlichen Lichtverhältnissen nicht sichtbaren Markierungsstoff, der jedoch bei Bestrahlung Fluoreszenz zeigt, in das der genamten Schicht entsprechende Überzugsmaterial integriert, und daß man die Gegenstände nach Aufbringen der genamten Schicht einer Bestrahlung aussetzt, und man das durch die Bestrahlung für jeden Gegenstand durch die den Markierungsstoff enthaltende Schicht abgegebene Signal verwendet, um die Verteilungsmerkmale in der genamten Schicht zu bestimmen, und das Sortieren der Gegenstände durchzuführen und/oder das Verfahren zur Ausbildung der Schicht auf den letztendlich der Beschichtung unterworfenen Gegenständen zu regeln.

2. Anwendung des Verfahrens nach Anspruch 1 bei der sogenamten "Bout-Froid"-Behandlung (Endstückkaltbehandlung) von Behältern aus Glas durch eine Behandlungskombination, die ein beigefügtes Polyäthylenwachs mit einem Molekulargewicht von etwa 2100 enthält, **dadurch gekennzeichnet,** daß 2,5-Bis-(5-Terbutylbenzoaxozolyl-2')-Thiophen in das Wachs eingebaut ist.

3. Anwendung des Verfahrens nach Anspruch 1 bei der Behandlung des oberen Randes des Mundstücks von Behältern durch ein Präparat, das die Heißversiegelung der Verschlußkappe des Behälters begünstigt, **dadurch gekennzeichnet,** daß ein Leuchtmittel in die Behandlungspräparat eingearbeitet wird, und daß der genannte obere Rand zur Kontrolle der Behandlung, zum Auffinden von Leckstellen und anderen möglichen Fehlstellen unter UV-Bestrahlung untersucht und dann gegebenenfalls die Behebung der Fehler sofort eingeleitet wird.

## Claims

1. Process for the coating of articles with one or more coating product coatings which are not visible under normal lighting conditions, characterized in that prior to carrying out a sorting of the articles, taking account of one of the characteristics of at least one of the said coatings, such as its thickness and/or an adjustment of the coating process with a view to obtaining the desired characteristic, into the coating product corresponding to the said coating is homogeneously incorporated prior to its deposition at least one marking agent which is not visible under normal lighting conditions, but which has a fluorescence and, following the deposition of said coating, the articles are subject to irradiation and the signal emitted by the coating containing the marking agent on each article under the effect of the irradiation is used for determining the distribution characteristics of said coating, followed by a sorting of said articles and/or the adjustment of the process for forming the said coating on the articles which are subsquently coated.

2. Application of the process according to claim 1 to "cold end" treatments of glass containers by a treatment composition containing an additive-containing polyethylene wax with a molecular weight of approximately 2100, characterized in that 2,5-bis(5 terbutyl-2'-benzoxazolyl)-thiophene is incorporated into the wax.

3. Application of the process according to claim 1 to the treatment of the upper edge of the rim of containers by a preparation aiding the heat sealing of their cap, characterized in that a fluorescent agent is introduced into said treatment preparation and that the upper edge is examined under ultraviolet radiation for checking the treatment, the detection of gaps or other possible imperfections, and, if appropriate, their correction.